# EUROPÄISCHE PATENTSCHRIFT

(11) **EP 2 198 789 B1**
(45) Veröffentlichungstag und Bekanntmachung des Hinweises auf die Patenterteilung: **14.12.2011**
(21) Anmeldenummer: 09013271.3
(22) Anmeldetag: 21.10.2009
(51) Int. Cl.: A61B 17/15

(54) **Instrument zum geführten Beginnen der Resektion der Kniescheibe während eines Eingriffs zum Einsetzen einer totalen Knieprothese**
Patellar saw cutting guide
Gabarit de coupe rotulienne

(30) Priorität: 21.10.2008 IT PO20080015
(43) Veröffentlichungstag der Anmeldung: 23.06.2010
(73) Patentinhaber: WALDEMAR LINK GmbH & Co. KG, 22339 Hamburg (DE)
(72) Erfinder: Baldini, Andrea, 59100 Prato PO (IT)
(74) Vertreter: Glawe, Delfs, Moll

(56) Entgegenhaltungen:
- WO-A1-2008/112996
- US-A- 5 021 055
- US-A1- 2003 212 403
- US-A1- 2008 097 450

## Beschreibung

Die Prothetisierung der Kniescheibe ist eine der verschiedenen Phasen des Eingriffs zum Einsetzen einer Knieprothese und besteht in der Resektion der abgenutzten Knorpelfläche, gemeinsam mit einem Teil des subchondralen Knochens, zur Erzeugung einer glatten Spongiosafläche, an der die Kniescheibenprothese, die von einem Polyethylenknopf verkörpert ist, mittels Zement verankert werden kann. Das Endkonstrukt einschließlich Polyethylenknopf darf nicht dicker als das ursprüngliche sein. Die Qualität der Resektion hängt von den Eigenschaften des verbleibenden Knochens ab, dessen Gesamtknochendicke nicht unter 12 bis 13 mm liegen darf und an allen vier Quadranten, oben, unten, medial und lateral, die gleiche Knochenqualität hinterlassen muss.

Die natürliche Form der Kniescheibe ist asymmetrisch, mit größerer medialer als lateraler Dicke, und das Schneideinstrument hat immer einen medialen Eintritt. Dies sorgt für eine Tendenz zum häufigen (die Literatur spricht von 7 bis 20 %) Erhalt einer asymmetrischen Resektion, typischerweise mit größerer medialer Restdicke und geringerer lateraler Dicke. Verschiedene biomechanische und klinische radiographische Untersuchungen haben gezeigt, dass eine mit einer hinsichtlich Dicke und/oder Symmetrie unangemessenen Resektion prothetisch ersetze Kniescheibe schlechtere kinematische Eigenschaften aufweist und Restschmerzen verursachen sowie gegebenenfalls einen erneuten Eingriff notwendig machen kann.

Derzeit kann die Resektion der Kniescheibe freihändig unter Verwendung einer oszillierenden Säge oder aber instrumentell mit Vorrichtungen durchgeführt werden, die dazu geschaffen sind, die Kniescheibe während der Resektion unbeweglich zu halten und ihre Resektion durch Schneiden oder Fräsen derart ausführen zu können, dass eine bekannte Gesamtrestknochendicke erzielt wird. Sowohl die freihändige als auch die instrumentelle Technik haben gute Ergebnisse hinsichtlich der Gewährleistung von Resektionen angemessener Dicke erzielt. Der Fehler, den es jedoch noch besser unter Kontrolle zu bekommen gilt, ist die Asymmetrie des Schnitts. Die existierenden Instrumente zur Führung des Kniescheibenschnitts weisen keine Referenzen für die beiden Gelenksflächen auf, die eine gleiche laterale und mediale Restknochendicke sicherstellen. Zudem sind diese Instrumente im Allgemeinen sehr sperrig und nicht einfach in der Verwendung.

Dokument US2008097450 A offenbart eine lehre gemäß dem Oberbegriff von Anspruch 1.

Erstrebenswert wäre demnach ein System, das die Schneideklinge in eine derartige Richtung führt, dass ein Knochenrest mit der gewünschten Dicke und symmetrisch in seinen Quadranten zurückgelassen wird.

Der Erfindung liegt die Aufgabe zu Grunde, den bis heute bestehenden Nachteil eines mangelhaft ausgeführten Schnitts in die Oberfläche der Kniescheibe für deren Prothetisierung auszuräumen. Die erfindungsgemäße Lösung liegt in den Merkmalen der unabhängigen Ansprüche. Vorteilhafte Weiterbildungen sind Gegenstand der abhängigen Ansprüche.

Bei einer Kniescheiben-Resektionslehre mit Auflagepunkten und einer Sägeführung ist erfingdungsgemäß vorgesehen, dass ein C-förmiger Hauptkörper mit Schenkeln und einem sie verbindenden Bogenstück vorgesehen ist, wobei an den Schenkeln aneinander gegenüberliegende Auflagepunkte zum Einspannen der Kniescheibe vorgesehen sind, und an dem Bogenstück ein Schneidschlitz als Führung angeordnet ist, der einen vorbestimmten Abstand von den Auflagepunkten an mindesten einem Schenkel aufweist.

Vorzugweise sind die Auflagepunkte an dem ersten Schenkel als zwei voneinander beabstandete Leisten gleicher Höhe ausgeführt. Weiter vorzugsweise ist der Auflagepunkt an dem zweiten Schenkel mit einem verbreiterten Kopf, insbesondere T-förmig ausgeführt, wobei er so dimensioniert ist, dass er sich mindestens über das von den Auflagepunkten an dem ersten Schenkel aufgespannte Gebiet erstreckt. Der Schneidschlitz ist vorzugsweise parallel zu der durch die unteren Auflagepunkte bestimmten Auflageebene ausgerichtet. Weiter vorzugsweise ist ein zweiter Schneidschlitz vorgesehen, der parallel zu dem ersten liegt. Die Schneidschlitze weisen zweckmäßigerweise einen Abstand von 12 bis 18 mm von den Auflagepunkten an dem ersten Schenkel auf, vorzugsweise der eine einen Abstand von 14 mm und der zweite Schneidschlitz einen Abstand von 16 mm.

Der Auflagepunkt an dem zweiten Schenkel ist vorzugsweise an einer Spanneinrichtung angeordnet, welche zur Anlage an einer Gelenkfläche der Kniescheibe ausgebildet ist. Die Spanneinrichtung ist vorzugsweise kanüliert und weist dazu eine Hohlbohrung und einen Durchmesser von vorzugsweise etwa 2 bis 5 mm auf.

An der Resektionslehre kann ein Handgriff angeordnet sein, und zwar insbesondere an dem Bogenstück. Der Handgriff ist vorzugsweise T-förmig zur besseren Handhabung.

Das Bogenstück ist vorzugsweise breiter als die Schenkel. Bewährt hat es sich, wenn es mindestens drei Mal breiter als der Schenkel ist, um so eine längere und damit genauere Führungsmöglichkeit zu geben.

Die Erfindung ermöglicht dank der wirksamen Steuerung der Position der Kniescheibe in Bezug auf das Schneideinstrument die Durchführung der Resektion der Kniescheibe während des Eingriffs zum Einsetzen einer totalen Knieprothese auf präzise, rasche und wiederholbare Weise.

Insbesondere wird dadurch die Symmetrie des verbleibenden Knochens nach der Durchführung der Resektion gewährleistet.

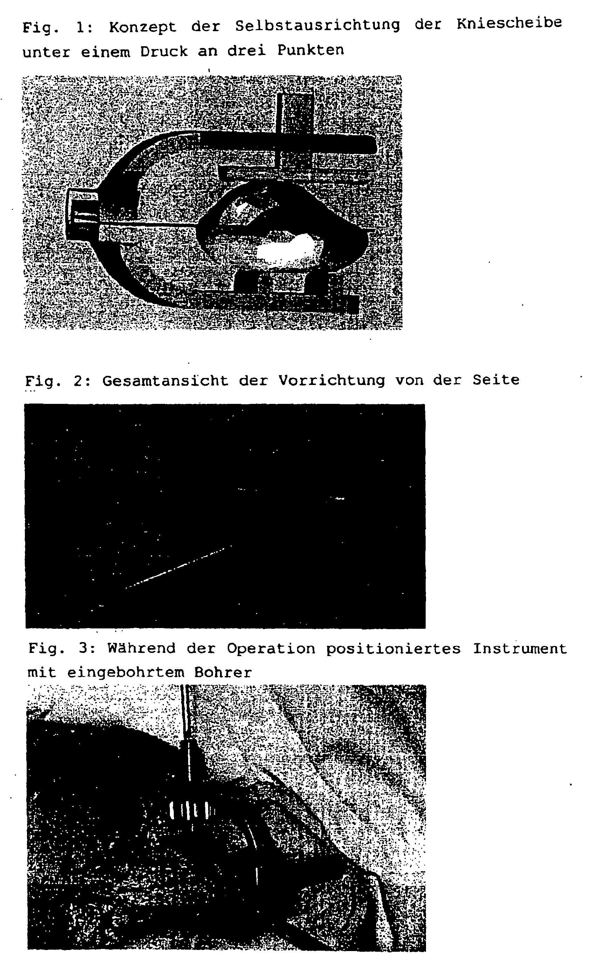

Die Erfindung ermöglicht die Positionierung der Kniescheibe in ihrer Umgebung und das Ausüben auf diese eines Drucks mit Schub an drei Punkten (Fig. 1), nämlich: der Gelenksfläche am Mittelgrat der Kniescheibe sowie dem medialen und lateralen Abschnitt der Nichtgelenksfläche der Kniescheibe. Der Kontakt an zwei Punkten, medial und lateral, der Nichtgelenksfläche wird durch zwei longitudinale Metallstäbe sichergestellt, die in Bezug auf den Arm des Instruments erhaben sind und zueinander um 2,5 cm beabstandet sind (Fig. 1 und 2). Ein doppelter seitlicher Schneideschlitz ermöglicht einen geführten Eintritt der Schneideklinge, was es mit der so positionierten Kniescheibe ermöglichen wird, die Resektion exakt parallel zur Nichtgelenksfläche der Kniescheibe auszuführen (Fig. 1, 2 und 3). Die im Instrument positionierte Kniescheibe wird mit dem horizontalen Stab komprimiert, der die Aufrechterhaltung der Spannung mittels eines Systems von Schraube mit Mutter ermöglicht (Fig. 3). Eine weitere Verstärkung der Fixierung ergibt sich aus der Verwendung eines Standardbohrers mit einem Durchmesser von 3,2 mm, der sich über 10 mm in die Kniescheibe bohrt und während des Schnitts im Sitz belassen wird.

Der Schnitt wird von einem der beiden Schlitze (mit der Standarddicke für Sägeblätter von chirurgischen oszillierenden Sägen von 1,27') des Instruments aus ausgeführt, je nachdem, ob eine Restknochenmenge von 14 oder 16 mm gewünscht ist (Fig. 1, 2 und 3, Übersicht 1). Der Schnitt wird durch den geführten Schlitz nur über etwa die Hälfte der Strecke ausgeführt, da das Instrument tatsächlich nur in der ersten Phase des Schnitts für Stabilität sorgt, während dann, wenn die Klinge den Mittelpunkt der Kniescheibe erreicht, die Kompression der Führung wegfällt. An diesem Punkt wird das Instrument abgenommen und der Schnitt manuell fertig gestellt, indem dem bereits mehr als ausreichend vorgegebenen Weg gefolgt wird.

Im Wesentlichen umfasst die Erfindung Folgendes:
- 1): einen "C"-förmigen Hauptkörper des Instruments mit T- förmigem Griff und zwei Schneideschlitzen (Fig. 2, Übersicht 1);
- 2): einen gleitbaren Stab mit Gegenmutter und kanülierter Schraubgleitführung, penetrierbar von einem Bohrer mit einem Durchmesser von 3,2 mm.

## Patentansprüche

1. Kniescheiben-Resektionslehre mit Auflagepunkten und einer Sägeführung mit einem C-förmigen Hauptkörper mit Schenkeln und einem sie verbindenden Bogenstück,
wobei an den Schenkeln aneinander gegenüberliegende Auflagepunkte zum Einspannen der Kniescheibe vorgesehen sind, und
an dem Bogenstück ein Schneidschlitz als Führung angeordnet ist, der einen vorbestimmten Abstand von den Auflagepunkten an mindesten einem Schenkel aufweist,
wobei an dem zweiten Schenkel eine Spanneinrichtung für den Auflagepunkt vorgesehen ist, die den Auflagepunkt in Richtung zu den Auflagepunkten an dem ersten Schenkel bewegt,
**dadurch gekennzeichnet, dass**
die Spanneinrichtung eine Bohrführung aufweist, die zu dem gegenüberliegenden Schenkel ausgerichtet ist.

2. Kniescheiben-Resektionslehre nach Anspruch 1,
**dadurch gekennzeichnet, dass**
an dem ersten Schenkel mindestens zwei Auflagepunkte vorgesehen sind, welche die Kniescheibe mit ihrer Nicht-Gelenkfläche in Bezug auf ihre Neigung ausrichtet.

3. Kniescheiben-Resektionslehre nach Anspruch 1,
**dadurch gekennzeichnet, dass**
die Spanneinrichtung eine Rücklaufsicherung umfasst.

4. Kniescheiben-Resektionslehre nach Anspruch 1,
**dadurch gekennzeichnet, dass**
die Spanneinrichtung als Gewindestange mit einer Haltemutter ausgeführt ist.

5. Kniescheiben-Resektionslehre nach Anspruch 1,
**dadurch gekennzeichnet, dass**
die Bohrführung als Hohlbohrung in der Gewindestange ausgeführt ist.

6. Kniescheiben-Resektionslehre nach Anspruch 5,
**dadurch gekennzeichnet, dass**
die Hohlbohrung einen Durchmesser von 2 bis 5 mm, vorzugsweise von 3,2 mm aufweist.

7. Kniescheiben-Resektionslehre nach einem der vorangehenden Ansprüche,
**dadurch gekennzeichnet, dass**
in einem Abstand von dem Schneidschlitz ein zweiter Schneidschlitz angeordnet ist.

8. Kniescheiben-Resektionslehre nach Anspruch 7,
**dadurch gekennzeichnet, dass**
die Schneidschlitze einen Abstand von 12 bis 18 mm von den Auflagepunkten an dem ersten Schenkel aufweisen, vorzugsweise von 14 bzw. 16 mm.

9. Kniescheiben-Resektionslehre nach einem der Ansprüche 1 bis 8,
**dadurch gekennzeichnet, dass**
die mindestens zwei Auflagepunkte des ersten Schenkels zusammen mit dem Auflagepunkt der Spanneinrichtung des zweiten Schenkels drei Auflagepunkte bilden.

## Claims

1. Kneecap resection gauge with support points and with a saw guide, with a C-shaped main body with brackets and an arch section connecting the brackets, wherein support points lying opposite one another are provided on the brackets for the purpose of clamping the kneecap, and a cutting slit is arranged as a guide on the arch section and is at a predetermined distance from the support points on at least one bracket, wherein a clamping mechanism for the support point is provided on the second bracket and moves the support point in the direction of the support points on the first bracket, **characterized in that** the clamping mechanism has a drill guide, which is oriented with respect to the opposite bracket.

2. Kneecap resection gauge according to Claim 1, **characterized in that** at least two support points are provided on the first bracket and orient the kneecap, with its non-articulating surface, with respect to the inclination thereof.

3. Kneecap resection gauge according to Claim 1, **characterized in that** the clamping mechanism comprises a back-run safety device.

4. Kneecap resection gauge according to Claim 1, **characterized in that** the clamping mechanism is designed as a threaded rod with a retaining nut.

5. Kneecap resection gauge according to Claim 1, **characterized in that** the drill guide is designed as a hollow bore in the threaded rod.

6. Kneecap resection gauge according to Claim 5, **characterized in that** the hollow bore has a diameter of 2 to 5 mm, preferably of 3.2 mm.

7. Kneecap resection gauge according to one of the preceding claims, **characterized in that**, at a distance from the cutting slit, there is a second cutting slit.

8. Kneecap resection gauge according to Claim 7, **characterized in that** the cutting slits are at a distance of 12 to 18 mm from the support points on the first bracket, preferably 14 and 16 mm, respectively.

9. Kneecap resection gauge according to one of Claims 1 to 8, **characterized in that** the at least two support points of the first bracket, together with the support point of the clamping mechanism of the second bracket, form three support points.

## Revendications

1. Gabarit de résection de la rotule présentant des points d'appui et un guide-scie doté d'un corps principal en forme de C présentant des branches reliées par une pièce arquée, des points d'appui mutuellement opposés étant prévus sur les branches pour serrer la rotule, une fente de découpe servant de guide étant disposée sur la pièce arquée et présentant une distance prédéterminée par rapport aux points d'appui d'au moins une branche, un dispositif de serrage du point d'appui étant prévu sur la deuxième branche et déplaçant le point d'appui en direction des points d'appui prévus sur la première branche, **caractérisé en ce que** le dispositif de serrage présente un guide d'alésage orienté vers la branche opposée.

2. Gabarit de résection de la rotule selon la revendication 1, **caractérisé en ce qu'**au moins deux points d'appui qui alignent l'inclinaison de la surface non d'articulation de la rotule sont prévus sur la première branche.

3. Gabarit de résection de la rotule selon la revendication 1, **caractérisé en ce que** le dispositif de serrage présente un blocage anti-recul.

4. Gabarit de résection de la rotule selon la revendication 1, **caractérisé en ce que** le dispositif de serrage est configuré comme tige filetée dotée d'un écrou de maintien.

5. Gabarit de résection de la rotule selon la revendication 1, **caractérisé en ce que** le guide d'alésage est configuré comme alésage creux ménagé dans la tige filetée.

6. Gabarit de résection de la rotule selon la revendication 5, **caractérisé en ce que** l'alésage creux présente un diamètre de 2 à 5 mm et de préférence de 3,2 mm.

7. Gabarit de résection de la rotule selon l'une des revendications précédentes, **caractérisé en ce qu'**une deuxième fente de découpe est disposée à distance de la fente de découpe.

8. Gabarit de résection de la rotule selon la revendication 7, **caractérisé en ce que** les fentes de découpe présentent une distance de 12 à 18 mm et de préférence de 14 ou de 16 mm par rapport aux points d'appui de la première branche.

9. Gabarit de résection de la rotule selon l'une des revendications 1 à 8, **caractérisé en ce que** les deux ou plusieurs points d'appui de la première branche forment trois points d'appui avec le point d'appui du dispositif de serrage de la deuxième branche.
